# EUROPEAN PATENT SPECIFICATION

(11) **EP 2 326 326 B1**
(45) Date of publication and mention of the grant of the patent: **09.10.2019**
(21) Application number: 09781825.6
(22) Date of filing: 13.08.2009
(51) Int. Cl.: A61K 31/40, A61K 31/4985, A61K 31/403, A61K 31/422, A61K 31/44, A61K 31/473, A61K 31/496, A61K 31/53, A61K 31/522, A61K 9/20, A61P 17/02, A61K 31/4196, A61K 9/00, A61K 31/513, A61K 31/5025, A61K 31/517, A61K 31/55, A61K 31/5513, A61K 31/553

(54) **DPP-4 INHIBITORS FOR USE FOR THE TREATMENT OF WOUND HEALING IN DIABETIC PATIENTS**
DPP-4 INHIBITOREN ZUR VERWENDUNG IN DER WUNDHEILUNG VON DIABETES-PATIENTEN
INHIBITEURS DE DPP-4 DESTINÉS À L'UTILISATION DANS LA CICATRISATION DES LÉSIONS DES DIABÉTIQUES

(30) Priority: 15.08.2008 WO PCT/EP2008/060740; 08.01.2009 EP 09150252; 19.05.2009 EP 09160682
(43) Date of publication of application: 01.06.2011
(73) Proprietor: Boehringer Ingelheim International GmbH, 55216 Ingelheim am Rhein (DE)
(72) Inventor: KLEIN, Thomas, 55216 Ingelheim am Rhein (DE); MARK, Michael, 55216 Ingelheim am Rhein (DE)
(74) Representative: Simon, Elke Anna Maria
(86) International application number: PCT/EP2009/060521
(87) International publication number: WO 2010/018217

(56) References cited:
- WO-A1-03/037327
- WO-A1-2004/050658
- WO-A1-2005/085246
- WO-A1-2006/005613
- WO-A1-2009/022010
- WO-A2-2004/018468
- WO-A2-2007/128761
- WO-A2-2008/131149
- DE-A1-102004 044 221
- US-A1- 2005 256 310
- US-A1- 2007 060 530
- US-A1- 2008 108 816
- THOMAS LEO ET AL: "(R)-8-(3-amino-piperidin-1-yl)-7-but-2-yn yl-3-methyl-1(4-methyl-quina zolin-2-ylmethyl)-3,7-dihydro-purine-2,6-d ione (BI 1356), a novel xanthine-based dipeptidyl peptidase 4 inhibitor, has a superior potency and longer duration of action compared with other dipeptidyl peptidase-4 inhibitors" JOURNAL OF PHARMACOLOGY AND EXPERIMENTAL THERAPEUTICS, AMERICAN SOCIETY FOR PHARMACOLOGY AND EXPERIMENTAL THERAPEUTICS, US, vol. 325, no. 1, 1 April 2008 (2008-04-01) , pages 175-182, XP009105508 ISSN: 0022-3565
- GWALTNEY S L: "Medicinal chemistry approaches to the inhibition of dipeptidyl peptidase IV" CURRENT TOPICS IN MEDICINAL CHEMISTRY, BENTHAM SCIENCE PUBLISHERS LTD, NL, vol. 8, no. 17, 1 January 2008 (2008-01-01), pages 1545-1552, XP002529044 ISSN: 1568-0266

## Description

The present invention relates to a DPP-4 inhibitor for use in wound healing in diabetic patients, wherein said DPP-4 inhibitor is selected from the group consisting of
1-[(4-methyl-quinazolin-2-yl)methyl]-3-methyl-7-(2-butyn-1-yl)-8-(3-(*R*)-amino-piperidin-1-yl)-xanthine,
1-[([1,5]naphthyridin-2-yl)methyl]-3-methyl-7-(2-butyn-1-yl)-8-((*R*)-3-amino-piperidin-1 -yl)-xanthine,
1-[(quinazolin-2-yl)methyl]-3-methyl-7-(2-butyn-1-yl)-8-((*R*)-3-amino-piperidin-1-yl)-xanthine, 2-((R)-3-amino-piperidin-1-yl)-3-(but-2-ynyl)-5-(4-methyl-quinazolin-2-ylmethyl)-3,5-dihydro-imidazo[4,5-d]pyridazin-4-one,
1-[(3-cyano-quinolin-2-yl)methyl]-3-methyl-7-(2-butyn-1-yl)-8-((*R*)-3-amino-piperidin-1-yl)-xanthine,
1-[(4-methyl-quinazolin-2-yl)methyl]-3-methyl-7-(2-butyn-1-yl)-8-[(*S*)-(2-amino-propyl)-methylamino]-xanthine,
1-[(4,6-dimethyl-pyrimidin-2-yl)methyl]-3-methyl-7-(2-butyn-1-yl)-8-((*R*)-3-amino-piperidin-1-yl)-xanthine and
1-[(quinoxalin-6-yl)methyl]-3-methyl-7-(2-butyn-1-yl)-8-((*R*)-3-amino-piperidin-1-yl)-xanthine, or a pharmaceutically acceptable salt thereof;
wherein said DPP-4 inhibitor is administered topically.

The present invention further relates to a topical preparation for use in wound healing in diabetic patients, said topical preparation comprising a DPP-4 inhibitor selected from the group consisting of
1-[(4-methyl-quinazolin-2-yl)methyl]-3-methyl-7-(2-butyn-1-yl)-8-(3-(*R*)-amino-piperidin-1-yl)-xanthine,
1-[([1,5]naphthyridin-2-yl)methyl]-3-methyl-7-(2-butyn-1-yl)-8-((*R*)-3-amino-piperidin-1 -yl)-xanthine,
1-[(quinazolin-2-yl)methyl]-3-methyl-7-(2-butyn-1-yl)-8-((*R*)-3-amino-piperidin-1-yl)-xanthine, 2-((R)-3-amino-piperidin-1-yl)-3-(but-2-ynyl)-5-(4-methyl-quinazolin-2-ylmethyl)-3,5-dihydro-imidazo[4,5-d]pyridazin-4-one,
1-[(3-cyano-quinolin-2-yl)methyl]-3-methyl-7-(2-butyn-1-yl)-8-((*R*)-3-amino-piperidin-1-yl)-xanthine,
1-[(4-methyl-quinazolin-2-yl)methyl]-3-methyl-7-(2-butyn-1-yl)-8-[(*S*)-(2-amino-propyl)-methylamino]-xanthine,
1-[(4,6-dimethyl-pyrimidin-2-yl)methyl]-3-methyl-7-(2-butyn-1-yl)-8-((*R*)-3-amino-piperidin-1-yl)-xanthine and
1-[(quinoxalin-6-yl)methyl]-3-methyl-7-(2-butyn-1-yl)-8-((*R*)-3-amino-piperidin-1-yl)-xanthine, or a pharmaceutically acceptable salt thereof;
with suitable carrier materials for topical preparations.

Reference is made to the present claims for further embodiments of the present invention.

The present disclosure relates to certain DPP-4 inhibitors for improving wound healing especially in diabetes patients (particularly type 2 diabetes patients), as well as to the use of these DPP-4 inhibitors for treating and/or preventing skin diseases, wounds and/or wound healing disturbances, in particular which are associated with diabetes. Pharmaceutical compositions and combinations for use in wound healing especially in diabetic patients comprising a DPP-4 inhibitor as defined herein optionally together with one or more other active substances are also contemplated.

Wound healing is essential for any organism to survive injury. Severe impairments in the wound healing process can lead to chronic wounds and finally to ulcers. Particularly, diabetes is associated with a disturbed wound healing process, such as e.g. slow healing of wounds or sores, chronic wounds and, finally, diabetes-associated ulcer (e.g. ulcus cruris arteriosum or necrobiosis lipoidica) or diabetic foot. Diabetic patients may face skin ulcerations with a lifetime risk of 15% to develop such complications which are often responsible for infections and amputations. A healthy skin repair process normally involves dynamic tissue movements including immune cell infiltration, angiogenesis, re-epithelialisation and remodelling. Moreover, it is now well established that wound inflammation is central to these processes and cruical for tissue regeneration.
Chronic wounds of diabetic patients demonstrate increased levels of metallo-proteases and produce less growth factor, essential for wound closure. Diabetic patients also often have peripherally vascular disease interfering with blood supply and capillary perfusion. In addition, neuropathy and lack of sensation in these patients may lead to deeper wounds and aggravation of the wound healing process. Controlling blood sugar is thereby a primary intervention for diabetic complications like impaired wound healing. However, because of the large number of complex physiological processes which are involved in wound healing, a great variety of factors can cause and influence disturbances of wound healing.

Diet therapy and exercise therapy are essential in the treatment of diabetes mellitus. When these therapies do not sufficiently control the conditions of patients (especially their blood sugar level), an oral or non-oral antidiabetic agent is additionally used for the treatment of diabetes. Conventional antidiabetic or antihyperglycemic agents include, without being limited to, metformin, sulphonylureas, thiazolidinediones, glinides, alpha-glucosidase blockers, GLP-1 and GLP-1 analogues, as well as insulin and insulin analogues. However, the use of these conventional antidiabetic or antihyperglycemic agents can be associated with various adverse effects. For example, metformin can be associated with lactic acidosis or gastrointestinal side effects; sulfonylureas, glinides and insulin or insulin analogues can be associated with hypoglycemia or weight gain; thiazolidinediones can be associated with edema, bone fracture, weight gain or heart failure/cardiac effects; and alpha-glucosidase blockers and GLP-1 or GLP-1 analogues can be associated with gastrointestinal adverse effects (e.g. dyspepsia, flatulence or diarrhea, or nausea or vomiting).

Further, the management of diabetes and its complications is complex and requires that many issues, beyond glycemic control, be addressed.

Thus, there is still a high unmet need and a high demand of novel and efficacious medicaments (advantageously antidiabetics) which positively affect wound healing especially in diabetic patients.

The enzyme DPP-4 also known as CD26 is a serine protease known to lead to the cleavage of a dipeptide from the N-terminal end of a number of proteins having at their N-terminal end a prolin or alanin residue. Due to this property DPP-4 inhibitors interfere with the plasma level of bioactive peptides including the peptide GLP-1 and are considered to be promising drugs for the treatment of diabetes mellitus.

For example, DPP-4 inhibitors and their uses, particularly their uses in metabolic (especially diabetic) diseases, are disclosed in WO 2002/068420, WO 2004/018467, WO 2004/018468, WO 2004/018469, WO 2004/041820, WO 2004/046148, WO 2005/051950, WO 2005/082906, WO 2005/063750, WO 2005/085246, WO 2006/027204, WO 2006/029769 or WO2007/014886; or in WO 2004/050658, WO 2004/111051, WO 2005/058901 or WO 2005/097798; or in WO 2006/068163, WO 2007/071738 or WO 2008/017670; or in WO 2007/128721 or WO 2007/128761.

As further DPP-4 inhibitors the following compounds can be mentioned:
- Sitagliptin (MK-0431) having the structural formula A below is (3*R*)-3-amino-1-[3-(trifluoromethyl)-5,6,7,8-tetrahydro-5H-[1,2,4]triazolo[4,3-a]pyrazin-7-yl]-4-(2,4,5-trifluorophenyl)butan-1-one, also named (2*R*)-4-oxo-4-[3-(trifluoromethyl)-5,6-dihydro[1,2,4]triazolo[4,3-a]pyrazin-7(8*H*)-yl]-1-(2,4,5-trifluorophenyl)butan-2-amine,

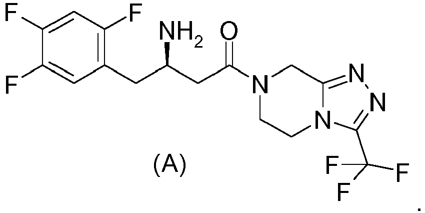

In one embodiment, sitagliptin is in the form of its dihydrogenphosphate salt, i.e. sitagliptin phosphate. In a further embodiment, sitagliptin phosphate is in the form of a crystalline anhydrate or monohydrate. A class of this embodiment refers to sitagliptin phosphate monohydrate. Sitagliptin free base and pharmaceutically acceptable salts thereof are disclosed in US Patent No. 6,699,871 and in Example 7 of WO 03/004498. Crystalline sitagliptin phosphate monohydrate is disclosed in WO 2005/003135 and in WO 2007/050485.
For details, e.g. on a process to manufacture, to formulate or to use this compound or a salt thereof, reference is thus made to these documents.

A tablet formulation for sitagliptin is commercially available under the trade name Januvia®. A tablet formulation for sitagliptin/metformin combination is commercially available under the trade name Janumet®.
- Vildagliptin (LAF-237) having the structural formula B below is (2S)-{[(3-hydroxyadamantan-1-yl)amino]acetyl}pyrrolidine-2-carbonitrile, also named (S)-1-[(3-hydroxy-1-adamantyl)amino]acetyl-2-cyano-pyrrolidine,

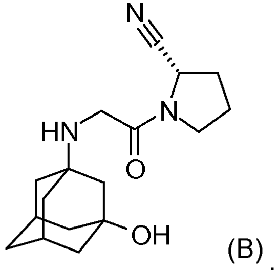

Vildagliptin is specifically disclosed in US Patent No. 6,166,063 and in Example 1 of WO 00/34241. Specific salts of vildagliptin are disclosed in WO 2007/019255. A crystalline form of vildagliptin as well as a vildagliptin tablet formulation are disclosed in WO 2006/078593. Vildagliptin can be formulated as described in WO 00/34241 or in WO 2005/067976. A modified release vildagliptin formulation is described in WO 2006/135723.
For details, e.g. on a process to manufacture, to formulate or to use this compound or a salt thereof, reference is thus made to these documents.

A tablet formulation for vildagliptin is commercially available under the trade name Galvus®. A tablet formulation for vildagliptin/metformin combination is commercially available under the trade name Eucreas®.
- Saxagliptin (BMS-477118) having the structural formula C below is (1S,3S,5S)-2-{(2S)-2-amino-2-(3-hydroxyadamantan-1-yl)acetyl}-2-azabicyclo[3.1.0]hexane-3-carbonitrile, also named (S)-3-hydroxyadamantylglycine-L-*cis*-4,5-methanoprolinenitrile,

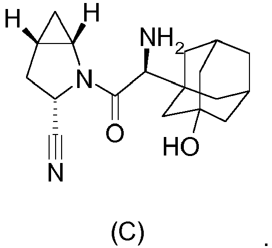

Saxagliptin is specifically disclosed in US Patent No. 6,395,767 and in Example 60 of WO 01/68603.

In one embodiment, saxagliptin is in the form of its HCI salt or its mono-benzoate salt as disclosed in WO 2004/052850. In a further embodiment, saxagliptin is in the form of the free base. In a yet further embodiment, saxagliptin is in the form of the monohydrate of the free base as disclosed in WO 2004/052850. Crystalline forms of the HCI salt and the free base of saxagliptin are disclosed in WO 2008/131149. A process for preparing saxagliptin is also disclosed in WO 2005/106011 and WO 2005/115982. Saxagliptin can be formulated in a tablet as described in WO 2005/117841.
For details, e.g. on a process to manufacture, to formulate or to use this compound or a salt thereof, reference is thus made to these documents.
- Alogliptin (SYR-322) having the structural formula E below is 2-({6-[(3R)-3-aminopiperidin-1-yl]-3-methyl-2,4-dioxo-3,4-dihydro-2H-pyrimidin-1-yl}methyl)benzonitrile

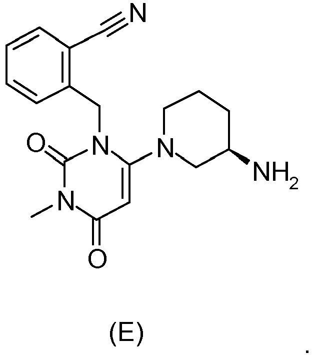

Alogliptin is specifically disclosed in US 2005/261271, EP 1586571 and in WO 2005/095381. In one embodiment, alogliptin is in the form of its benzoate salt, its hydrochloride salt or its tosylate salt each as disclosed in WO 2007/035629. A class of this embodiment refers to alogliptin benzoate. Polymorphs of alogliptin benzoate are disclosed in WO 2007/035372. A process for preparing alogliptin is disclosed in WO 2007/112368 and, specifically, in WO 2007/035629. Alogliptin (namely its benzoate salt) can be formulated in a tablet and administered as described in WO 2007/033266. Formulations of aloglipitin with metformin or pioglitazone are described in WO 2008/093882 or WO 2009/011451, respectively.
For details, e.g. on a process to manufacture, to formulate or to use this compound or a salt thereof, reference is thus made to these documents.
- (2*S*)-1-{[2-(5-Methyl-2-phenyl-oxazol-4-yl)-ethylamino]-acetyl}-pyrrolidine-2-carbonitrile or a pharmaceutically acceptable salt thereof, preferably the mesylate, or (2*S*)-1-{[1,1,-Dimethyl-3-(4-pyridin-3-yl-imidazol-1-yl)-propylamino]-acetyl}-pyrrolidine-2-carbonitrile or a pharmaceutically acceptable salt thereof:
   These compounds and methods for their preparation are disclosed in WO 03/037327.
   The mesylate salt of the former compound as well as crystalline polymorphs thereof are disclosed in WO 2006/100181. The fumarate salt of the latter compound as well as crystalline polymorphs thereof are disclosed in WO 2007/071576. These compounds can be formulated in a pharmaceutical composition as described in WO 2007/017423.
   For details, e.g. on a process to manufacture, to formulate or to use these compounds or salts thereof, reference is thus made to these documents.
- (*S*)-1-((2*S*,3*S*,11b*S*)-2-Amino-9,10-dimethoxy-1,3,4,6,7,11b-hexahydro-2H-pyrido[2,1-a]isoquinolin-3-yl)-4-fluoromethyl-pyrrolidin-2-one or a pharmaceutically acceptable salt thereof:

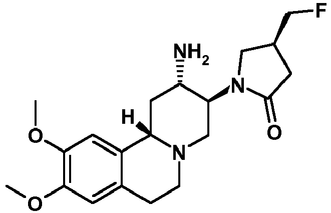

This compound and methods for its preparation are disclosed in WO 2005/000848. A process for preparing this compound (specifically its dihydrochloride salt) is also disclosed in WO 2008/031749, WO 2008/031750 and WO 2008/055814. This compound can be formulated in a pharmaceutical composition as described in WO 2007/017423.
For details, e.g. on a process to manufacture, to formulate or to use this compound or a salt thereof, reference is thus made to these documents.
- (3,3-Difluoropyrrolidin-1-yl)-((2S,4S)-4-(4-(pyrimidin-2-yl)piperazin-1-yl)pyrrolidin-2-yl)methanone (also named gosogliptin) or a pharmaceutically acceptable salt thereof:
   This compound and methods for its preparation are disclosed in WO 2005/116014 and US 7291618.

For details, e.g. on a process to manufacture, to formulate or to use this compound or a salt thereof, reference is thus made to these documents.
- (1((3S,4S)-4-amino-1-(4-(3,3-difluoropyrrolidin-1-yl)-1,3,5-triazin-2-yl)pyrrolidin-3-yl)-5,5-difluoropiperidin-2-one or a pharmaceutically acceptable salt thereof:

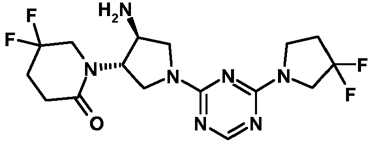

This compound and methods for its preparation are disclosed in WO 2007/148185 and US 20070299076. For details, e.g. on a process to manufacture, to formulate or to use this compound or a salt thereof, reference is thus made to these documents.
- (2S,4S)-1-{2-[(3S,1R)-3-(1H-1,2,4-Triazol-1-ylmethyl)cyclopentylamino]-acetyl}-4-fluoropyrrolidine-2-carbonitrile (also named melogliptin) or a pharmaceutically acceptable salt thereof:

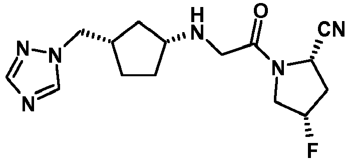

This compound and methods for its preparation are disclosed in WO 2006/040625 and WO 2008/001195. Specifically claimed salts include the methanesulfonate and p-toluenesulfonate. For details, e.g. on a process to manufacture, to formulate or to use this compound or a salt thereof, reference is thus made to these documents.
- (R)-2-[6-(3-Amino-piperidin-1-yl)-3-methyl-2,4-dioxo-3,4-dihydro-2H-pyrimidin-1-ylmethyl]-4-fluoro-benzonitrile or a pharmaceutically acceptable salt thereof:

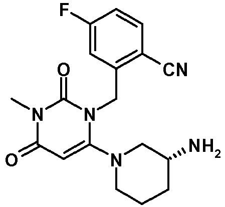

This compound and methods for its preparation and use are disclosed in WO 2005/095381, US 2007060530, WO 2007/033350, WO 2007/035629, WO 2007/074884, WO 2007/112368, WO 2008/114807, WO 2008/114800 and WO 2008/033851. Specifically claimed salts include the succinate (WO 2008/067465), benzoate, benzenesulfonate, p-toluenesulfonate, (R)-mandelate and hydrochloride. For details, e.g. on a process to manufacture, to formulate or to use this compound or a salt thereof, reference is thus made to these documents.
- 5-{(S)-2-[2-((S)-2-Cyano-pyrrolidin-1-yl)-2-oxo-ethylamino]-propyl}-5-(1H-tetrazol-5-yl)-10,11-dihydro-5H-dibenzo[a,d]cycloheptene-2,8-dicarboxylic acid bis-dimethylamide or a pharmaceutically acceptable salt thereof:

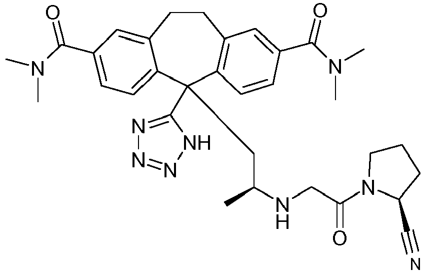

This compound and methods for its preparation are disclosed in WO 2006/116157 and US 2006/270701. For details, e.g. on a process to manufacture, to formulate or to use this compound or a salt thereof, reference is thus made to these documents.
- 3-{(2S,4S)-4-[4-(3-Methyl-1-phenyl-1H-pyrazol-5-yl)piperazin-1-yl]pyrrolidin-2-ylcarbonyl}thiazolidine (also named teneligliptin) or a pharmaceutically acceptable salt thereof:
   This compound and methods for its preparation are disclosed in WO 02/14271. Specific salts are disclosed in WO 2006/088129 and WO 2006/118127 (including hydrochloride, hydrobromide, inter alia). Combination therapy using this compound is described in WO 2006/129785. For details, e.g. on a process to manufacture, to formulate or to use this compound or a salt thereof, reference is thus made to these documents.
- [(2R)-1-{[(3R)-pyrrolidin-3-ylamino]acetyl}pyrrolidin-2-yl]boronic acid (also named dutogliptin) or a pharmaceutically acceptable salt thereof:
   This compound and methods for its preparation are disclosed in WO 2005/047297, WO 2008/109681 and WO 2009/009751. Specific salts are disclosed in WO 2008/027273 (including citrate, tartrate). A formulation of this compound is described in WO 2008/144730. For details, e.g. on a process to manufacture, to formulate or to use this compound or a salt thereof, reference is thus made to these documents.
- (2S,4S)-1-[2-[(4-ethoxycarbonylbicyclo[2.2.2]oct-1-yl)amino]acetyl]-4-fluoropyrrolidine-2-carbonitrile or a pharmaceutically acceptable salt thereof:
   This compound and methods for its preparation are disclosed in WO 2005/075421, US 2008/146818 and WO 2008/114857. For details, e.g. on a process to manufacture, to formulate or to use this compound or a salt thereof, reference is thus made to these documents.
- 2-({6-[(3R)-3-amino-3-methylpiperidin-1-yl]-1,3-dimethyl-2,4-dioxo-1,2,3,4-tetrahydro-5H-pyrrolo[3,2-d]pyrimidin-5-yl}methyl)-4-fluorobenzonitrile or a pharmaceutically acceptable salt thereof, or 6-[(3R)-3-amino-piperidin-1-yl]-5-(2-chloro-5-fluoro-benzyl)-1,3-dimethyl-1,5-dihydro-pyrrolo[3,2-d]pyrimidine-2,4-dione or a pharmaceutically acceptable salt thereof:
   These compounds and methods for their preparation are disclosed in WO 2009/084497 and WO 2006/068163, respectively. For details, e.g. on a process to manufacture, to formulate or to use these compounds or salts thereof, reference is thus made to these documents.

Within the scope of the present disclosure it has now surprisingly been found that certain DPP-4 inhibitors as defined herein have surprising and particularly advantageous properties, which make them particularly suitable for use in wound healing, especially in diabetic patients (particularly in type 2 diabetes patients).

Thus, the present disclosure provides a DPP-4 inhibitor as defined herein for use in healing of diabetic or non-diabetic wounds.

The present disclosure further provides a DPP-4 inhibitor as defined herein for promoting or improving wound healing in diabetic and non-diabetic patients, especially in diabetic patients.

The present disclosure further provides a DPP-4 inhibitor as defined herein for use in the treatment and/or prevention (including preventing or slowing the progression or reducing the occurrence or delaying the onset) of wound healing deficit or impairments in the wound healing process, especially in diabetic patients.

The present disclosure further provides a DPP-4 inhibitor as defined herein for treating and/or preventing (including preventing or slowing the progression or reducing the occurrence or delaying the onset) of skin diseases, wounds and/or wound healing disturbances including, but not limited to, those which are associated with diabetes.

The present disclosure further provides a DPP-4 inhibitor as defined herein for use in the treatment and/or prevention (including preventing or slowing the progression or reducing the occurrence or delaying the onset) of chronic skin ulcerations, wounds or sores, destructive wound inflammation (e.g. infiltration of neutrophils), delayed or impaired wound healing or closure, or disturbed tissue regeneration, formation or remodeling, especially in diabetic patients.

The present disclosure further provides a DPP-4 inhibitor as defined herein for diminishing wound size and/or for improving wound closure, particularly of diabetes-associated wounds.

The present disclosure further provides a DPP-4 inhibitor as defined herein for improving wound epithelialization, wound morphology and/or tissue regeneration, particularly of diabetes-associated wounds.

The present disclosure further provides a DPP-4 inhibitor as defined herein for promoting neo-or re-epithelialization, particularly of diabetes-associated wounds.

The present disclosure further provides a DPP-4 inhibitor as defined herein for reducing destructive wound inflammation, such as e.g. for reducing the number of polymorphonuclear neutrophils (PMN), particularly in diabetes-associated wounds.

The present disclosure further provides a DPP-4 inhibitor as defined herein for treating and/or preventing (including reducing the risk of developing or progressing) metabolic disorders or diseases, especially diabetes (particularly type 2 diabetes), in patients with or at risk of skin diseases, wounds and/or wound healing disturbances or impairments (in particular which are associated with diabetes), such as e.g. those described herein (e.g. chronic skin ulcerations, wounds or sores, destructive wound inflammation (e.g. infiltration of neutrophils), delayed or impaired wound healing or closure, or disturbed tissue regeneration, formation or remodeling).

Further, according to another aspect of the disclosure, there is provided the use of a DPP-4 inhibitor as defined herein for the manufacture of a medicament for one or more of the following purposes:
- preventing, slowing the progression of, delaying or treating a metabolic disorder or disease, such as e.g. type 1 diabetes mellitus, type 2 diabetes mellitus, impaired glucose tolerance (IGT), impaired fasting blood glucose (IFG), hyperglycemia, postprandial hyperglycemia, overweight, obesity, dyslipidemia, hyperlipidemia, hypercholesterolemia, hypertension, atherosclerosis, endothelial dysfunction, osteoporosis, chronic systemic inflammation, retinopathy, neuropathy, nephropathy and/or metabolic syndrome;
- improving glycemic control and/or for reducing of fasting plasma glucose, of postprandial plasma glucose and/or of glycosylated hemoglobin HbA1c;
- preventing, slowing, delaying or reversing progression from impaired glucose tolerance (IGT), impaired fasting blood glucose (IFG), insulin resistance and/or from metabolic syndrome to type 2 diabetes mellitus;
- preventing, reducing the risk of, slowing the progression of, delaying or treating of complications of diabetes mellitus such as micro- and macrovascular diseases, such as nephropathy, micro- or macroalbuminuria, proteinuria, retinopathy, cataracts, neuropathy, learning or memory impairment, neurodegenerative or cognitive disorders, cardio- or cerebrovascular diseases, tissue ischaemia, diabetic foot or ulcus, atherosclerosis, hypertension, endothelial dysfunction, myocardial infarction, acute coronary syndrome, unstable angina pectoris, stable angina pectoris, peripheral arterial occlusive disease, cardiomyopathy, heart failure, heart rhythm disorders, vascular restenosis, and/or stroke;
- reducing body weight or preventing an increase in body weight or facilitating a reduction in body weight;
- preventing, slowing, delaying or treating the degeneration of pancreatic beta cells and/or the decline of the functionality of pancreatic beta cells and/or for improving and/or restoring the functionality of pancreatic beta cells and/or stimulating and/or restoring the functionality of pancreatic insulin secretion;
- preventing, slowing, delaying or treating non alcoholic fatty liver disease (NAFLD) including hepatic steatosis, non-alcoholic steatohepatitis (NASH) and/or liver fibrosis;
- preventing, slowing the progression of, delaying or treating type 2 diabetes with primary or secondary failure to conventional (oral) antihyperglycemic mono- or combination therapy;
- achieving a reduction in the dose of conventional antihyperglycemic medication required for adequate therapeutic effect;
- reducing the risk for adverse effects associated with conventional (oral or non-oral) antihyperglycemic medication; and/or
- maintaining and/or improving the insulin sensitivity and/or for treating or preventing hyperinsulinemia and/or insulin resistance;
particularly in a patient having (or being at risk of) a skin disease, wound and/or wound healing disturbance or impairment, in particular associated with diabetes, such as e.g. any of those wound and/or skin disorders described herein (e.g. chronic skin ulcerations, wounds or sores, destructive wound inflammation (e.g. infiltration of neutrophils), delayed or impaired wound healing or closure, or disturbed tissue regeneration, formation or remodeling); optionally in combination with one or more other active substances, such as e.g. any of those mentioned herein.

The present disclosure further provides a pharmaceutical composition for use in wound healing, especially in diabetic patients, said pharmaceutical composition comprising a DPP-4 inhibitor as defined herein and optionally one or more pharmaceutically acceptable carriers and/or diluents.

The present disclosure further provides a fixed or non-fixed combination including a kit-of-parts for use in wound healing, especially in diabetic patients, said combination comprising a DPP-4 inhibitor as defined herein and one or more other active substances, e.g. any of those mentioned herein.

The present disclosure further provides the use of a DPP-4 inhibitor as defined herein optionally in combination with one or more other active substances, such as e.g. any of those mentioned herein, for the manufacture of a pharmaceutical composition for wound healing, especially in diabetic patients.

The present disclosure further provides a pharmaceutical composition for use in wound healing, especially in diabetic patients, said pharmaceutical composition comprising a DPP-4 inhibitor as defined herein and optionally one or more other active substances, such as e.g. any of those mentioned herein, such as e.g. for separate, sequential, simultaneous, concurrent or chronologically staggered use of the active ingredients.

The present disclosure further provides a method of wound healing, especially in diabetic patients, said method comprising administering to a subject in need thereof (particularly a human patient) an effective amount of a DPP-4 inhibitor as defined herein, optionally alone or in combination, such as e.g. separately, sequentially, simultaneously, concurrently or chronologically staggered with an effective amount of one, two or more other active substances, such as e.g. any of those mentioned herein.

An embodiment of the DPP-4 inhibitors according to this disclosure refers to those DPP-4 inhibitors which - besides their glycemic action - exert direct favourable (e.g. extraglycemic) effects on the wound repairing process in a type 2 diabetic subject. Beyond improving glycemic control, these DPP-4 inhibitors are suitable for providing additional therapeutic benefits to patients with or at risk of skin diseases, wounds and/or wound healing disturbances or impairments.

An embodiment of the patients described herein, particularly of those having (or being at risk of) skin diseases, wounds and/or wound healing disturbances or impairment, in particular associated with diabetes, being amenable to the therapies of this disclosure includes, without being limited to, diabetes patients for whom metformin therapy is inappropriate due to intolerability or contraindication against metformin and/or who have renal disease, renal dysfunction, or insufficiency or impairment of renal function (including patients having chronic renal insufficiency, such as e.g. patients with mild, moderate or severe renal impairment or with end stage renal disease, and/or having nephropathy, micro- or macroalbuminuria, or proteinuria).

Other aspects of the present disclosure become apparent to the skilled person from the foregoing and following remarks.

A DPP-4 inhibitor within the meaning of the present disclosure includes, without being limited to, any of those DPP-4 inhibitors mentioned hereinabove and hereinbelow, preferably orally active DPP-4 inhibitors.

In a first embodiment (embodiment **A**), a DPP-4 inhibitor in the context of the present disclosure is any DPP-4 inhibitor of 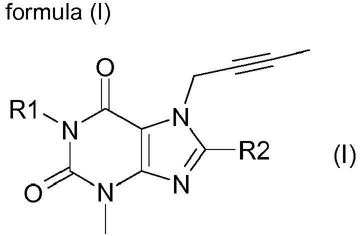 or 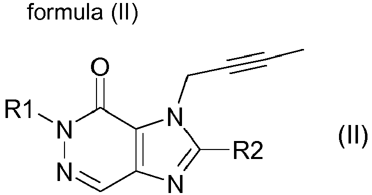 or 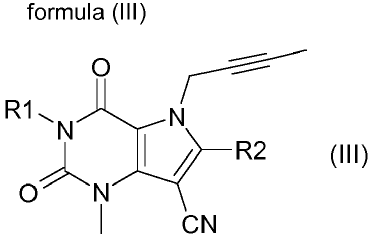 or 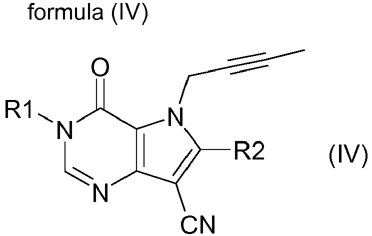 wherein **R1** denotes ([1,5]naphthyridin-2-yl)methyl, (quinazolin-2-yl)methyl, (quinoxalin-6-yl)methyl, (4-methyl-quinazolin-2-yl)methyl, 2-cyano-benzyl, (3-cyano-quinolin-2-yl)methyl, (3-cyano-pyridin-2-yl)methyl, (4-methyl-pyrimidin-2-yl)methyl, or (4,6-dimethyl-pyrimidin-2-yl)methyl and **R2** denotes 3-(*R*)-amino-piperidin-1-yl, (2-amino-2-methyl-propyl)-methylamino or (2-(*S*)-amino-propyl)-methylamino,
or its pharmaceutically acceptable salt.

In a second embodiment (embodiment **B**), a DPP-4 inhibitor in the context of the present disclosure is a DPP-4 inhibitor selected from the group consisting of
sitagliptin, vildagliptin, saxagliptin, alogliptin,
(2*S*)-1-{[2-(5-Methyl-2-phenyl-oxazol-4-yl)-ethylamino]-acetyl}-pyrrolidine-2-carbonitrile,
(2*S*)-1-{[1,1,-Dimethyl-3-(4-pyridin-3-yl-imidazol-1-yl)-propylamino]-acetyl}-pyrrolidine-2-carbonitrile,
(*S*)-1-((2*S*,3*S*,11b*S*)-2-Amino-9,10-dimethoxy-1,3,4,6,7,11b-hexahydro-2H-pyrido[2,1-a]isoquinolin-3-yl)-4-fluoromethyl-pyrrolidin-2-one,
(3,3-Difluoropyrrolidin-1-yl)-((2S,4S)-4-(4-(pyrimidin-2-yl)piperazin-1-yl)pyrrolidin-2-yl)methanone,
(1((3S,4S)-4-amino-1-(4-(3,3-difluoropyrrolidin-1-yl)-1,3,5-triazin-2-yl)pyrrolidin-3-yl)-5,5-difluoropiperidin-2-one,
(2S,4S)-1-{2-[(3S,1R)-3-(1H-1,2,4-Triazol-1-ylmethyl)cyclopentylamino]-acetyl}-4-fluoropyrrolidine-2-carbonitrile,
(R)-2-[6-(3-Amino-piperidin-1-yl)-3-methyl-2,4-dioxo-3,4-dihydro-2H-pyrimidin-1-ylmethyl]-4-fluoro-benzonitrile,
5-{(S)-2-[2-((S)-2-Cyano-pyrrolidin-1-yl)-2-oxo-ethylamino]-propyl}-5-(1H-tetrazol-5-yl)-10,11-dihydro-5H-dibenzo[a,d]cycloheptene-2,8-dicarboxylic acid bis-dimethylamide,
3-{(2S,4S)-4-[4-(3-Methyl-1-phenyl-1H-pyrazol-5-yl)piperazin-1-yl]pyrrolidin-2-ylcarbonyl}thiazolidine,
[(2R)-1-{[(3R)-pyrrolidin-3-ylamino]acetyl}pyrrolidin-2-yl]boronic acid,
(2S,4S)-1-[2-[(4-ethoxycarbonylbicyclo[2.2.2]oct-1-yl)amino]acetyl]-4-fluoropyrrolidine-2-carbonitrile,
2-({6-[(3R)-3-amino-3-methyipiperidin-1-yl]-1,3-dimethyl-2,4-dioxo-1,2,3,4-tetrahydro-5H-pyrrolo[3,2-d]pyrimidin-5-yl}methyl)-4-fluorobenzonitrile, and
6-[(3R)-3-amino-piperidin-1-yl]-5-(2-chloro-5-fluoro-benzyl)-1,3-dimethyl-1,5-dihydro-pyrrolo[3,2-d]pyrimidine-2,4-dione,
or its pharmaceutically acceptable salt.

Regarding the first embodiment (embodiment **A**), preferred DPP-4 inhibitors are any or all of the following compounds and their pharmaceutically acceptable salts:
- 1-[(4-methyl-quinazolin-2-yl)methyl]-3-methyl-7-(2-butyn-1-yl)-8-(3-(*R*)-amino-piperidin-1-yl)-xanthine (compare WO 2004/018468, example 2(142)): 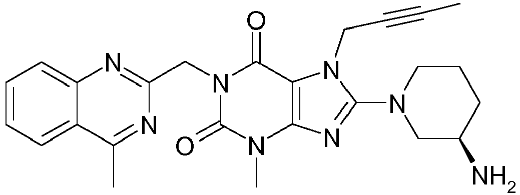
- 1-[([1,5]naphthyridin-2-yl)methyl]-3-methyl-7-(2-butyn-1-yl)-8-((*R*)-3-amino-piperidin-1 - yl)-xanthine (compare WO 2004/018468, example 2(252)): 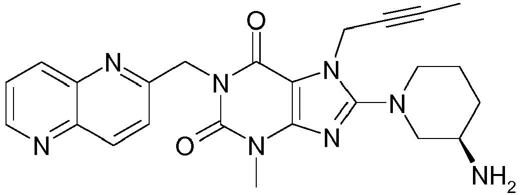
- 1-[(Quinazolin-2-yl)methyl]-3-methyl-7-(2-butyn-1-yl)-8-((*R*)-3-amino-piperidin-1-yl)-xanthine (compare WO 2004/018468, example 2(80)): 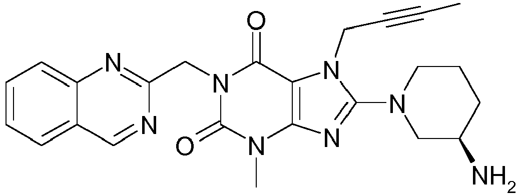
- 2-((R)-3-Amino-piperidin-1-yl)-3-(but-2-yinyl)-5-(4-methyl-quinazolin-2-ylmethyl)-3,5-dihydro-imidazo[4,5-d]pyridazin-4-one (compare WO 2004/050658, example 136): 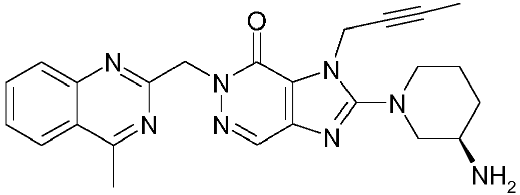
- 1-[(4-Methyl-quinazolin-2-yl)methyl]-3-methyl-7-(2-butyin-1-yl)-8-[(2-amino-2-methylpropyl)-methylamino]-xanthine (compare WO 2006/029769, example 2(1)): 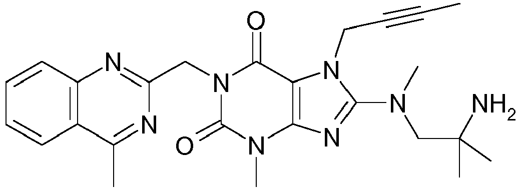
- 1-[(3-Cyano-quinolin-2-yl)methyl]-3-methyl-7-(2-butyn-1-yl)-8-((*R*)-3-amino-piperidin-1- yl)-xanthine (compare WO 2005/085246, example 1(30)): 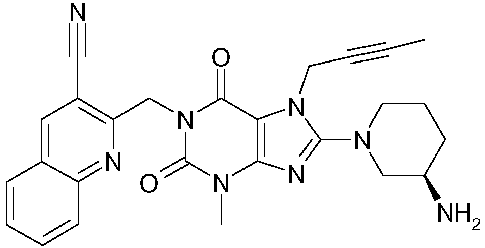
- 1-(2-Cyano-benzyl)-3-methyl-7-(2-butyn-1-yl)-8-((*R*)-3-amino-piperidin-1-yl)-xanthine (compare WO 2005/085246, example 1(39)): 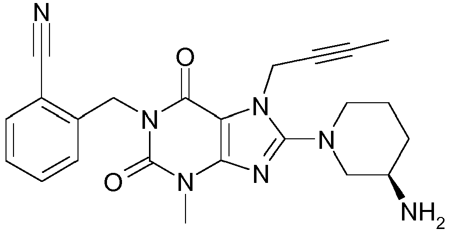
- 1-[(4-Methyl-quinazolin-2-yl)methyl]-3-methyl-7-(2-butyn-1-yl)-8-[(*S*)-(2-amino-propyl)-methylamino]-xanthine (compare WO 2006/029769, example 2(4)): 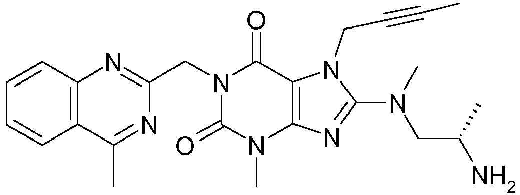
- 1-[(3-Cyano-pyridin-2-yl)methyl]-3-methyl-7-(2-butyn-1-yl)-8-((*R*)-3-amino-piperidin-1-yl)-xanthine (compare WO 2005/085246, example 1(52)): 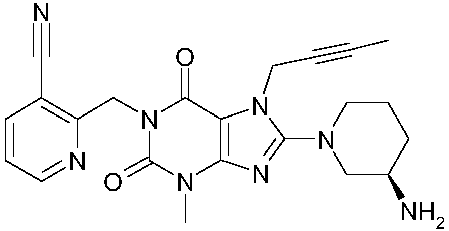
- 1-[(4-Methyl-pyrimidin-2-yl)methyl]-3-methyl-7-(2-butyn-1-yl)-8-((*R*)-3-amino-piperidin-1 - yl)-xanthine (compare WO 2005/085246, example 1(81)): 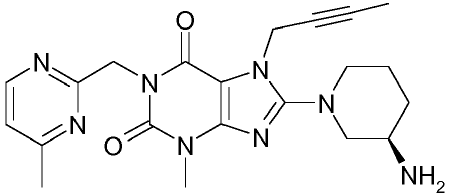
- 1-[(4,6-Dimethyl-pyrimidin-2-yl)methyl]-3-methyl-7-(2-butyn-1-yl)-8-((*R*)-3-amino-piperidin-1-yl)-xanthine (compare WO 2005/085246, example 1(82)): 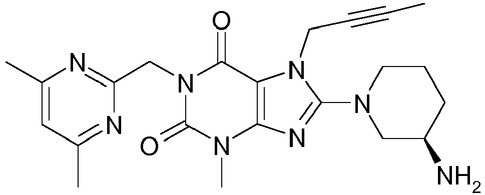
- 1-[(Quinoxalin-6-yl)methyl]-3-methyl-7-(2-butyn-1-yl)-8-((*R*)-3-amino-piperidin-1-yl)-xanthine (compare WO 2005/085246, example 1(83)):

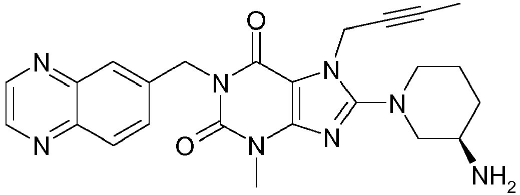

These DPP-4 inhibitors are distinguished from structurally comparable DPP-4 inhibitors, as they combine exceptional potency and a long-lasting effect with favourable pharmacological properties, receptor selectivity and a favourable side-effect profile or bring about unexpected therapeutic advantages or improvements when combined with other pharmaceutical active substances. Their preparation is disclosed in the publications mentioned.

A more preferred DPP-4 inhibitor among the abovementioned DPP-4 inhibitors of embodiment **A** of this disclosure is 1-[(4-methyl-quinazolin-2-yl)methyl]-3-methyl-7-(2-butyn-1-yl)-8-(3-(*R*)-amino-piperidin-1-yl)-xanthine, particularly the free base thereof (which is also known as BI 1356).

Unless otherwise noted, according to this disclosure it is to be understood that the definitions of the active compounds (including the DPP-4 inhibitors) mentioned hereinabove and hereinbelow also comprise their pharmaceutically acceptable salts as well as hydrates, solvates and polymorphic forms thereof. With respect to salts, hydrates and polymorphic forms thereof, particular reference is made to those which are referred to herein.

With respect to embodiment **A**, the methods of synthesis for the DPP-4 inhibitors according to embodiment **A** of this disclosure are known to the skilled person. Advantageously, the DPP-4 inhibitors according to embodiment **A** of this disclosure can be prepared using synthetic methods as described in the literature. Thus, for example, purine derivatives of formula (I) can be obtained as described in WO 2002/068420, WO 2004/018468, WO 2005/085246, WO 2006/029769 or WO 2006/048427.
Purine derivatives of formula (II) can be obtained as described, for example, in WO 2004/050658 or WO 2005/110999.
Purine derivatives of formula (III) and (IV) can be obtained as described, for example, in WO 2006/068163, WO 2007/071738 or WO 2008/017670. The preparation of those DPP-4 inhibitors, which are specifically mentioned hereinabove, is disclosed in the publications mentioned in connection therewith. Polymorphous crystal modifications and formulations of particular DPP-4 inhibitors are disclosed in WO 2007/128721 and WO 2007/128724, respectively. Formulations of particular DPP-4 inhibitors with metformin or other combination partners are described in PCT/EP2009053978. Typical dosage strengths of the dual combination of BI 1356 / metformin are 2.5/500 mg, 2.5/850 mg and 2.5/1000 mg

With respect to embodiment **B**, the methods of synthesis for the DPP-4 inhibitors of embodiment **B** are described in the scientific literature and/ or in published patent documents, particularly in those cited herein.

For pharmaceutical application in warm-blooded vertebrates, particularly humans, the compounds of this disclosure are usually used in dosages from 0.001 to 100 mg/kg body weight, preferably at 0.1-15 mg/kg, in each case 1 to 4 times a day. For this purpose, the compounds, optionally combined with other active substances, may be incorporated together with one or more inert conventional carriers and/or diluents, e.g. with corn starch, lactose, glucose, microcrystalline cellulose, magnesium stearate, polyvinylpyrrolidone, citric acid, tartaric acid, water, water/ethanol, water/glycerol, water/sorbitol, water/polyethylene glycol, propylene glycol, cetylstearyl alcohol, carboxymethylcellulose or fatty substances such as hard fat or suitable mixtures thereof into conventional galenic preparations such as plain or coated tablets, capsules, powders, suspensions or suppositories.

The pharmaceutical compositions according to this disclosure comprising the DPP-4 inhibitors as defined herein are thus prepared by the skilled person using pharmaceutically acceptable formulation excipients as described in the art. Examples of such excipients include, without being restricted to diluents, binders, carriers, fillers, lubricants, flow promoters, crystallisation retardants, disintegrants, solubilizers, colorants, pH regulators, surfactants and emulsifiers.

In a certain embodiment, a DPP-4 inhibitor of the disclosure may be for oral use and thus maybe in the form of a tablet. Such a tablet typically comprises the active ingredient(s) with one or more diluents, fillers and/or carriers, and, optionally, one or more binders, one or more lubricants, one or more disintegrants, and/or one or more glidants, as well as, if desired, a film overcoat.

Examples of suitable diluents for compounds according to embodiment **A** include cellulose powder, calcium hydrogen phosphate, erythritol, low substituted hydroxypropyl cellulose, mannitol, pregelatinized starch or xylitol. Among those diluents mannitol, low substituted hydroxypropyl cellulose and pregelatinized starch are to be emphasized.

Examples of suitable lubricants for compounds according to embodiment **A** include talc, polyethyleneglycol, calcium behenate, calcium stearate, hydrogenated castor oil or magnesium stearate. Among those lubricants magnesium stearate is to be emphasized.

Examples of suitable binders for compounds according to embodiment **A** include copovidone (copolymerisates of vinylpyrrolidon with other vinylderivates), hydroxypropyl methylcellulose (HPMC), hydroxypropylcellulose (HPC), polyvinylpyrrolidon (povidone), pregelatinized starch, or low-substituted hydroxypropylcellulose (L-HPC). Among those binders copovidone and pregelatinized starch are to be emphasized.

Examples of suitable disintegrants for compounds according to embodiment **A** include corn starch or crospovidone. Among those disintegrants corn starch is to be emphasized.

Suitable methods of preparing pharmaceutical formulations of the DPP-4 inhibitors according to embodiment **A** of the disclosure are
- direct tabletting of the active substance in powder mixtures with suitable tabletting excipients;
- granulation with suitable excipients and subsequent mixing with suitable excipients and subsequent tabletting as well as film coating; or
- packing of powder mixtures or granules into capsules.

Suitable granulation methods are
- wet granulation in the intensive mixer followed by fluidised bed drying;
- one-pot granulation;
- fluidised bed granulation; or
- dry granulation (e.g. by roller compaction) with suitable excipients and subsequent tabletting or packing into capsules.

An exemplary composition preferably for oral use (particularly a tablet) of a DPP-4 inhibitor according to embodiment **A** of the disclosure comprises the first diluent mannitol, pregelatinized starch as a second diluent with additional binder properties, the binder copovidone, the disintegrant corn starch, and magnesium stearate as lubricant; wherein copovidone and/or corn starch may be optional.

In another embodiment, a DPP-4 inhibitor of the disclosure may be for topic use and thus e.g. in the form of an ointment. Such a topical preparation typically comprises the active ingredient(s) with suitable carrier materials for topical preparations, such as, for example, glycerides, semi- synthetic and synthetic glycerides, hydrogenated oils, liquid waxes, liquid paraffins, liquid fatty alcohols, sterols, polyethylene glycols and/or cellulose derivatives.

For details on dosage forms, formulations and administration of DPP-4 inhibitors of this disclosure, reference is made to scientific literature and/ or published patent documents, particularly to those cited herein.

With respect to the first embodiment (embodiment **A**), the dosage typically required of the DPP-4 inhibitors mentioned herein in embodiment **A** when administered intravenously is 0.1 mg to 10 mg, preferably 0.25 mg to 5 mg, and when administered orally is 0.5 mg to 100 mg, preferably 2.5 mg to 50 mg or 0.5 mg to 10 mg, more preferably 2.5 mg to 10 mg or 1 mg to 5 mg, in each case 1 to 4 times a day. Thus, e.g. the dosage of 1-[(4-methyl-quinazolin-2-yl)methyl]-3-methyl-7-(2-butyn-1-yl)-8-(3-(R)-amino-piperidin-1-yl)-xanthine when administered orally is 0.5 mg to 10 mg per patient per day, preferably 2.5 mg to 10 mg or 1 mg to 5 mg per patient per day.

A dosage form prepared with a pharmaceutical composition comprising a DPP-4 inhibitor mentioned herein in embodiment **A** contain the active ingredient in a dosage range of 0.1-100 mg. Thus, e.g. particular dosage strengths of 1-[(4-methyl-quinazolin-2-yl)methyl]-3-methyl-7-(2-butyn-1-yl)-8-(3-(R)-amino-piperidin-1-yl)-xanthine are 0.5 mg, 1 mg, 2.5 mg, 5 mg and 10 mg.

With respect to the second embodiment (embodiment **B**), the doses of DPP-4 inhibitors mentioned herein in embodiment **B** to be administered to mammals, for example human beings, of, for example, approximately 70 kg body weight, may be generally from about 0.5 mg to about 350 mg, for example from about 10 mg to about 250 mg, preferably 20-200 mg, more preferably 20-100 mg, of the active moiety per person per day, or from about 0.5 mg to about 20 mg, preferably 2.5-10 mg, per person per day, divided preferably into 1 to 4 single doses which may, for example, be of the same size. Single dosage strengths comprise, for example, 10, 25, 40, 50, 75, 100, 150 and 200 mg of the DPP-4 inhibitor active moiety.

A dosage strength of the DPP-4 inhibitor sitagliptin is usually between 25 and 200 mg of the active moiety. A recommended dose of sitagliptin is 100 mg calculated for the active moiety (free base anhydrate) once daily. Unit dosage strengths of sitagliptin free base anhydrate (active moiety) are 25, 50, 75, 100, 150 and 200 mg. Particular unit dosage strengths of sitagliptin (e.g. per tablet) are 25, 50 and 100 mg. An equivalent amount of sitagliptin phosphate monohydrate to the sitagliptin free base anhydrate is used in the pharmaceutical compositions, namely, 32.13, 64.25, 96.38, 128.5, 192.75, and 257 mg, respectively. Adjusted dosages of 25 and 50 mg sitagliptin are used for patients with renal failure. Typical dosage strengths of the dual combination of sitagliptin / metformin are 50/500 mg and 50/1000 mg.

A dosage range of the DPP-4 inhibitor vildagliptin is usually between 10 and 150 mg daily, in particular between 25 and 150 mg, 25 and 100 mg or 25 and 50 mg or 50 and 100 mg daily. Particular examples of daily oral dosage are 25, 30, 35, 45, 50, 55, 60, 80, 100 or 150 mg. In a more particular aspect, the daily administration of vildagliptin may be between 25 and 150 mg or between 50 and 100 mg. In another more particular aspect, the daily administration of vildagliptin may be 50 or 100 mg. The application of the active ingredient may occur up to three times a day, preferably one or two times a day. Particular dosage strengths are 50 mg or 100 mg vildagliptin. Typical dosage strengths of the dual combination of vildagliptin / metformin are 50/850 mg and 50/1000 mg.

Alogliptin may be administered to a patient at a daily dose of between 5 mg/day and 250 mg/day, optionally between 10 mg and 200 mg, optionally between 10 mg and 150 mg, and optionally between 10 mg and 100 mg of alogliptin (in each instance based on the molecular weight of the free base form of alogliptin). Thus, specific dosage amounts that may be used include, but are not limited to 10 mg, 12.5 mg, 20 mg, 25 mg, 50 mg, 75 mg and 100 mg of alogliptin per day. Alogliptin may be administered in its free base form or as a pharmaceutically acceptable salt form.

Saxagliptin may be administered to a patient at a daily dose of between 2.5 mg/day and 100 mg/day, optionally between 2.5 mg and 50 mg. Specific dosage amounts that may be used include, but are not limited to 2.5 mg, 5 mg, 10 mg, 15 mg, 20 mg, 30 mg , 40 mg, 50 mg and 100 mg of saxagliptin per day. Typical dosage strengths of the dual combination of saxagliptin / metformin are 2.5/500 mg and 2.5/1000 mg.

A special embodiment of the DPP-4 inhibitors of this disclosure refers to those orally administered DPP-4 inhibitors which are therapeutically efficacious at low dose levels, e.g. at oral dose levels < 100 mg or < 70 mg per patient per day, preferably < 50 mg, more preferably < 30 mg or < 20 mg, even more preferably from 1 mg to 10 mg, particularly from 1 mg to 5 mg (more particularly 5 mg), per patient per day (if required, divided into 1 to 4 single doses, particularly 1 or 2 single doses, which may be of the same size, preferentially, administered orally once- or twice daily (more preferentially once-daily), advantageously, administered at any time of day, with or without food. Thus, for example, the daily oral amount 5 mg BI 1356 can be given in a once daily dosing regimen (i.e. 5 mg BI 1356 once daily) or in a twice daily dosing regimen (i.e. 2.5 mg BI 1356 twice daily), at any time of day, with or without food.

A particularly preferred DPP-4 inhibitor to be emphasized within the meaning of this disclosure is 1-[(4-methyl-quinazolin-2-yl)methyl]-3-methyl-7-(2-butyn-1-yl)-8-(3-(*R*)-amino-piperidin-1-yl)-xanthine (also known as BI 1356). BI 1356 exhibits high potency, 24h duration of action, and a wide therapeutic window. In patients with type 2 diabetes receiving multiple oral doses of 1, 2.5, 5 or 10 mg of BI 1356 once daily for 12 days, BI 1356 shows favourable pharmacodynamic and pharmacokinetic profile (see e.g. Table 1 below) with rapid attainment of steady state (e.g. reaching steady state plasma levels (> 90% of the pre-dose plasma concentration on Day 13) between second and fifth day of treatment in all dose groups), little accumulation (e.g. with a mean accumulation ratio R_{A,AUC} ≤ 1.4 with doses above 1 mg) and preserving a long-lasting effect on DPP-4 inhibition (e.g. with almost complete (> 90%) DPP-4 inhibition at the 5 mg and 10 mg dose levels, i.e. 92.3 and 97.3% inhibition at steady state, respectively, and > 80% inhibition over a 24h interval after drug intake), as well as significant decrease in 2h postprandial blood glucose excursions by ≥ 80 % (already on Day 1) in doses ≥ 2.5 mg, and with the cumulative amount of unchanged parent compound excreted in urine on Day 1 being below 1% of the administered dose and increasing to not more than about 3-6% on Day 12 (renal clearance CL_{R,ss} is from about 14 to about 70 mL/min for the administered oral doses, e.g. for the 5 mg dose renal clearance is about 70 ml/min). In people with type 2 diabetes BI 1356 shows a placebo-like safety and tolerability. With low doses of about ≥ 5 mg, BI 1356 acts as a true once-daily oral drug with a full 24 h duration of DPP-4 inhibition. At therapeutic oral dose levels, BI 1356 is mainly excreted via the liver and only to a minor extent (about < 7% of the administered oral dose) via the kidney. BI 1356 is primarily excreted unchanged via the bile. The fraction of BI 1356 eliminated via the kidneys increases only very slightly over time and with increasing dose, so that there will likely be no need to modify the dose of BI 1356 based on the patients' renal function. The non-renal elimination of BI 1356 in combination with its low accumulation potential and broad safety margin may be of significant benefit in a patient population that has a high prevalence of renal insufficiency and diabetic nephropathy.

**Table 1: Geometric mean (gMean) and geometric coefficient of variation (gCV) of pharmacokinetic parameters of BI 1356 at steady state (Day 12)**

| **Parameter** | **1 mg gMean (gCV)** | **2.5 mg gMean (gCV)** | **5 mg gMean (gCV)** | **10 mg gMean (gCV)** |
|---|---|---|---|---|
| AUC₀₋₂₄ [nmol·h/L] | 40.2 (39.7) | 85.3 (22.7) | 118 (16.0) | 161 (15.7) |
| AUC_{T,SS} [nmol·h/L] | 81.7 (28.3) | 117 (16.3) | 158 (10.1) | 190 (17.4) |
| Cₘₐₓ [nmol/L] | 3.13 (43.2) | 5.25 (24.5) | 8.32 (42.4) | 9.69 (29.8) |
| C_{max,ss} [nmol/L] | 4.53 (29.0) | 6.58 (23.0) | 11.1 (21.7) | 13.6 (29.6) |
| tₘₐₓ* [h] | 1.50 [1.00 - 3.00] | 2.00 [1.00 - 3.00] | 1.75 [0.92 - 6.02] | 2.00 [1.50 - 6.00] |
| t_{max,ss}* [h] | 1.48 [1.00 - 3.00] | 1.42 [1.00 - 3.00] | 1.53 [1.00 - 3.00] | 1.34 [0.50 - 3.00] |
| T_{½,ss} [h] | 121 (21.3) | 113 (10.2) | 131 (17.4) | 130 (11.7) |
| Accumulation t_{½,} [h] | 23.9(44.0) | 12.5(18.2) | 11.4 (37.4) | 8.59 (81.2) |
| R_{A,Cmax} | 1.44 (25.6) | 1.25 (10.6) | 1.33 (30.0) | 1.40 (47.7) |
| R_{A,AUC} | 2.03 (30.7) | 1.37 (8.2) | 1.33 (15.0) | 1.18 (23.4) |
| fe₀₋₂₄ [%] | NC | 0.139 (51.2) | 0.453 (125) | 0.919 (115) |
| fe_{T,SS} [%] | 3.34 (38.3) | 3.06 (45.1) | 6.27 (42.2) | 3.22 (34.2) |
| CL_{R,ss} [mL/min] | 14.0 (24.2) | 23.1 (39.3) | 70 (35.0) | 59.5 (22.5) |

| | | | | |
|---|---|---|---|---|
| * median and range [min-max] NC not calculated as most values below lower limit of quantification | | | | |

As different metabolic functional disorders often occur simultaneously, it is quite often indicated to combine a number of different active principles with one another. Thus, depending on the functional disorders diagnosed, improved treatment outcomes may be obtained if a DPP-4 inhibitor is combined with active substances customary for the respective disorders, such as e.g. one or more active substances selected from among the other antidiabetic substances, especially active substances that lower the blood sugar level or the lipid level in the blood, raise the HDL level in the blood, lower blood pressure or are indicated in the treatment of atherosclerosis or obesity.

Further, within the meaning of this disclosure, optionally additionally to other combination partners, a DPP-4 inhibitor may be combined with one or more drugs typically used for treating (chronic) wounds.

The DPP-4 inhibitors mentioned above - besides their use in mono-therapy - may also be used in conjunction with other active substances, by means of which improved treatment results can be obtained. Such a combined treatment may be given as a free combination of the substances or in the form of a fixed combination, for example in a tablet or capsule. Pharmaceutical formulations of the combination partner(s) needed for this may either be obtained commercially as pharmaceutical compositions or may be formulated by the skilled man using conventional methods. The active substances which may be obtained commercially as pharmaceutical compositions are described in numerous places in the prior art, for example in the list of drugs that appears annually, the "Rote Liste ®" of the federal association of the pharmaceutical industry, or in the annually updated compilation of manufacturers' information on prescription drugs known as the "Physicians' Desk Reference".

Examples of antidiabetic combination partners are metformin; sulphonylureas such as glibenclamide, tolbutamide, glimepiride, glipizide, gliquidon, glibornuride and gliclazide; nateglinide; repaglinide; thiazolidinediones such as rosiglitazone and pioglitazone; PPAR gamma modulators such as metaglidases; PPAR-gamma agonists such as GI 262570; PPAR-gamma antagonists; PPAR-gamma/alpha modulators such as tesaglitazar, muraglitazar, aleglitazar, indeglitazar, AVE0897 and KRP297; PPAR-gamma/alpha/delta modulators; AMPK-activators such as AICAR; acetyl-CoA carboxylase (ACC1 and ACC2) inhibitors; diacylglycerol-acetyltransferase (DGAT) inhibitors; pancreatic beta cell GCRP agonists such as SMT3-receptor-agonists and GPR119; 11β-HSD-inhibitors; FGF19 agonists or analogues; alpha-glucosidase blockers such as acarbose, voglibose and miglitol; alpha2-antagonists; insulin and insulin analogues such as human insulin, insulin lispro, insulin glusilin, r-DNA-insulinaspart, NPH insulin, insulin detemir, insulin zinc suspension and insulin glargin; Gastric inhibitory Peptide (GIP); pramlintide, davalintide; amylin and amylin analogues or GLP-1 and GLP-1 analogues such as Exendin-4, e.g. exenatide, exenatide LAR, liraglutide, taspoglutide, AVE-0010, LY-2428757, LY-2189265, semaglutide or albiglutide; SGLT2-inhibitors such as KGT-1251; inhibitors of protein tyrosine-phosphatase; inhibitors of glucose-6-phosphatase; fructose-1,6-bisphosphatase modulators; glycogen phosphorylase modulators; glucagon receptor antagonists; phosphoenolpyruvatecarboxykinase (PEPCK) inhibitors; pyruvate dehydrogenasekinase (PDK) inhibitors; inhibitors of tyrosine-kinases (50 mg to 600 mg) such as PDGF-receptor-kinase (cf. EP-A-564409, WO 98/35958, US 5093330, WO 2004/005281, and WO 2006/041976); glucokinase/regulatory protein modulators incl. glucokinase activators; glycogen synthase kinase inhibitors; inhibitors of the SH2-domain-containing inositol 5-phosphatase type 2 (SHIP2) ; IKK inhibitors such as high-dose salicylate ; JNK1 inhibitors ; protein kinase C-theta inhibitors; beta 3 agonists such as ritobegron, YM 178, solabegron, talibegron, N-5984, GRC-1087, rafabegron, FMP825; aldosereductase inhibitors such as AS 3201, zenarestat, fidarestat, epalrestat, ranirestat, NZ-314, CP-744809, and CT-112; SGLT-1 or SGLT-2 inhibitors, such as e.g. dapagliflozin, sergliflozin, atigliflozin, larnagliflozin or canagliflozin (or the compound of formula (I-S) or (I-K) from WO 2009/035969); KV 1.3 channel inhibitors; GPR40 modulators; SCD-1 inhibitors; CCR-2 antagonists; dopamine receptor agonists (bromocriptine mesylate [Cycloset]); and other DPP IV inhibitors.

Metformin is usually given in doses varying from about 250 mg to 3000 mg, particularly from about 500 mg to 2000 mg up to 2500 mg per day using various dosing regimens, such as e.g. from about 100 mg to 500 mg or 200 mg to 850 mg (1-3 times a day), or about 300 mg to 1000 mg once or twice a day, or delayed-release metformin in doses of about 100 mg to 1000 mg or preferably 500 mg to 1000 mg once or twice a day or about 500 mg to 2000 mg once a day. Particular dosage strengths may be 250, 500, 625, 750, 850 and 1000 mg of metformin hydrochloride.

A dosage of pioglitazone is usually of about 1-10 mg, 15 mg, 30 mg, or 45 mg once a day.

Rosiglitazone is usually given in doses from 4 to 8 mg once (or divided twice) a day (typical dosage strengths are 2, 4 and 8 mg).

Glibenclamide (glyburide) is usually given in doses from 2.5 to 20 mg once (or divided twice) a day (typical dosage strengths are 1.25, 2.5 and 5 mg), or micronized glibenclamide in doses from 0.75 to 12 mg once (or divided twice) a day (typical dosage strengths are 1.5, 3, 4.5 and 6 mg).

Glipizide is usually given in doses from 2.5 to 40 mg once (or divided twice) a day (typical dosage strengths are 5 and 10 mg), or extended-release glipizide in doses from 5 to 20 mg once a day (typical dosage strengths are 2.5, 5 and 10 mg).

Glimepiride is usually given in doses from 1 to 8 mg once a day (typical dosage strengths are 1, 2 and 4 mg).

A dual combination of glibenclamide/metformin is usually given in doses from 1.25/250 once daily to 10/1000 mg twice daily (typical dosage strengths are 1.25/250, 2.5/500 and 5/500 mg).
A dual combination of glipizide/metformin is usually given in doses from 2.5/250 to 10/1000 mg twice daily (typical dosage strengths are 2.5/250, 2.5/500 and 5/500 mg).
A dual combination of glimepiride/metformin is usually given in doses from 1/250 to 4/1000 mg twice daily.
A dual combination of rosiglitazone/glimepiride is usually given in doses from 4/1 once or twice daily to 4/2 mg twice daily (typical dosage strengths are 4/1, 4/2, 4/4, 8/2 and 8/4 mg).
A dual combination of pioglitazone/glimepiride is usually given in doses from 30/2 to 30/4 mg once daily (typical dosage strengths are 30/4 and 45/4 mg).
A dual combination of rosiglitazone/metformin is usually given in doses from 1/500 to 4/1000 mg twice daily (typical dosage strengths are 1/500, 2/500, 4/500, 2/1000 and 4/1000 mg).
A dual combination of pioglitazone/metformin is usually given in doses from 15/500 once or twice daily to 15/850 mg thrice daily (typical dosage strengths are 15/500 and 15/850 mg).

The non-sulphonylurea insulin secretagogue nateglinide is usually given in doses from 60 to 120 mg with meals (up to 360 mg/day, typical dosage strengths are 60 and 120 mg); repaglinide is usually given in doses from 0.5 to 4 mg with meals (up to 16 mg/day, typical dosage strengths are 0.5, 1 and 2 mg). A dual combination of repaglinide/metformin is available in dosage strengths of 1/500 and 2/850 mg.

Acarbose is usually given in doses from 25 to 100 mg with meals (up to 300 mg/day, typical dosage strengths are 25, 50 and 100 mg). Miglitol is usually given in doses from 25 to 100 mg with meals (up to 300 mg/day, typical dosage strengths are 25, 50 and 100 mg).

Conventional antidiabetics and antihyperglycemics typically used in mono- or dual or triple (add-on or initial) combination therapy may include, without being limited to, metformin, sulphonylureas, thiazolidinediones, glinides, alpha-glucosidase blockers, GLP-1 and GLP-1 analogues, as well as insulin and insulin analogues, such as e.g. those agents indicated herein by way of example, including combinations thereof.

Examples of combination partners that lower the lipid level in the blood are HMG-CoA-reductase inhibitors such as simvastatin, atorvastatin, lovastatin, fluvastatin, pravastatin, pitavastatin and rosuvastatin; fibrates such as bezafibrate, fenofibrate, clofibrate, gemfibrozil, etofibrate and etofyllinclofibrate; nicotinic acid and the derivatives thereof such as acipimox; PPAR-alpha agonists; PPAR-delta agonists; inhibitors of acyl-coenzyme A:cholesterolacyltransferase (ACAT; EC 2.3.1.26) such as avasimibe; cholesterol resorption inhibitors such as ezetimib; substances that bind to bile acid, such as cholestyramine, colestipol and colesevelam; inhibitors of bile acid transport; HDL modulating active substances such as D4F, reverse D4F, LXR modulating active substances and FXR modulating active substances; CETP inhibitors such as torcetrapib, JTT-705/dalcetrapib, anacetrapib or compound 12 from WO 2007/005572 (anacetrapib); LDL receptor modulators; and ApoB100 antisense RNA.

A dosage of atorvastatin is usually from 1 mg to 40 mg or 10 mg to 80 mg once a day

Examples of combination partners that lower blood pressure are beta-blockers such as atenolol, bisoprolol, celiprolol, metoprolol and carvedilol; diuretics such as hydrochlorothiazide, chlortalidon, xipamide, furosemide, piretanide, torasemide, spironolactone, eplerenone, amiloride and triamterene; calcium channel blockers such as amlodipine, nifedipine, nitrendipine, nisoldipine, nicardipine, felodipine, lacidipine, lercanipidine, manidipine, isradipine, nilvadipine, verapamil, gallopamil and diltiazem; ACE inhibitors such as ramipril, lisinopril, cilazapril, quinapril, captopril, enalapril, benazepril, perindopril, fosinopril and trandolapril; as well as angiotensin II receptor blockers (ARBs) such as telmisartan, candesartan, valsartan, losartan, irbesartan, olmesartan and eprosartan.

A dosage of telmisartan is usually from 20 mg to 320 mg or 40 mg to 160 mg per day.

Examples of combination partners which increase the HDL level in the blood are Cholesteryl Ester Transfer Protein (CETP) inhibitors; inhibitors of endothelial lipase; regulators of ABC1; LXRalpha antagonists; LXRbeta agonists; PPAR-delta agonists; LXRalpha/beta regulators, and substances that increase the expression and/or plasma concentration of apolipoprotein A-I.

Examples of combination partners for the treatment of obesity are sibutramine; tetrahydrolipstatin (orlistat), cetilistat; alizyme; dexfenfluramine; axokine; cannabinoid receptor 1 antagonists such as the CB1 antagonist rimonobant; MCH-1 receptor antagonists; MC4 receptor agonists; NPY5 as well as NPY2 antagonists; beta3-AR agonists such as SB-418790 and AD-9677; 5HT2c receptor agonists such as APD 356 (Iorcaserin); myostatin inhibitors; Acrp30 and adiponectin; steroyl CoA desaturase (SCD1) inhibitors; fatty acid synthase (FAS) inhibitors; CCK receptor agonists; Ghrelin receptor modulators; Pyy 3-36; orexin receptor antagonists; and tesofensine; as well as the dual combinations bupropion/naltrexone, bupropion/zonisamide, topiramate/phentermine and pramlintide/metreleptin.

Examples of combination partners for the treatment of atherosclerosis are phospholipase A2 inhibitors; inhibitors of tyrosine-kinases (50 mg to 600 mg) such as PDGF-receptor-kinase (cf. EP-A-564409, WO 98/35958, US 5093330, WO 2004/005281, and WO 2006/041976); oxLDL antibodies and oxLDL vaccines; apoA-1 Milano; ASA; and VCAM-1 inhibitors.

Examples of drugs typically used for treating (chronic) wounds include, without being limited, orally and topically applied agents, such as e.g. pentoxifylline, iloprost, antimicrobials (such as e.g. iodine based preparations, silver releasing agents, antimicrobial agents which target bacteria at several levels, systemic antibiotics or the like), glyceryl trinitrate (nitric oxide donor), calcium antagonists (such as diltiazem or nifedipine), systemic corticosteroids, zinc (applied topically or orally), phenytoin (applied topically), retinoids, and/or analgesics.

The present disclosure is not to be limited in scope by the specific embodiments described herein. Various modifications of the disclosure in addition to those described herein may become apparent to those skilled in the art from the present disclosure. Such modifications are intended to fall within the scope of the appended claims.

Further embodiments, features and advantages of the present disclosure may become apparent from the following examples. The following examples serve to illustrate, by way of example, the principles of the disclosure without restricting it.

### Examples

The ob/ob mice have been used and are accepted as experimental model for diabetes-impaired wound healing. These animals suffer from severe diabetes and obesity syndromes which show similar characteristics like in the human situation (e.g. obesity, insulin resistance). Insulin and diet reduce hyperglycemia in obese mice just they as they do in obese and diabetic subjects, but neither improves healing in these animals as has been shown recently.

Ob/ob mice treated for 12 day with BI 1356 show a marked improvement of wound re-epithelialization upon treatment, as the distance between wound margin epithelia appears significantly reduced (BI 1356: 0.74 ±0.90 mm; control: 2.02 ±1.07 mm, A, B). In accordance to improved wound epithelialization, we observe an overall accumulation of PMN in impaired wounds of control but not in BI 1356 *ob*/*ob* mice. The glucose excursion following an oral glucose tolerance assay is reduced by 25% (absolute AUC) for BI 1356 treated animals (C).

In summary, BI 1356 demonstrates surprisingly acceleration of wound healing in ob/ob mice which correlates with a decreased glucose excursion. This effects could be, at least, partially accountable to its blood sugar lowering capacity. Therefore, the DPP-4 inhibitor BI 1356 could be used for further diabetic complication associated with wound healing like the diabetic foot.

### Methods

Female C57BI/6J-ob/ob mice (Charles River, Sulzfeld) in the age of 8-9 weeks were used. 6 excisional wounds (including the panniculus carnosus) were placed on the back of each animal under ketamin anaesthesia. The animals were treated daily with 3 mg/kg of BI 1356 or methycellulose (1-2%). On day 10 an oral glucose tolerance test (2g/kg) was performed (0, 30, 60, 90, 120 und 180 min) at the tail tip. Animals were killed under isofluran anaesthesia by cervical dislocation and wounds were excised for histological analysis. Serum was further taken for the detection of DPP-4 activity. Histology was performed in 6-8 µm paraffin embedded and paraformaldehyd fixed cuttings with eosin-hematoxylin staining. Moreover, polymorphonuclear neutrophils (PMN) were identified by immunohistological staining of Ly6G as a robust readout for wound inflammation. (Ref. S. Frank, Methods in Molecular Medicine 2003, Kämpfer, Diabetes 2006)

### Results

### A) Histology of wounds with and without BI 1356 treatment

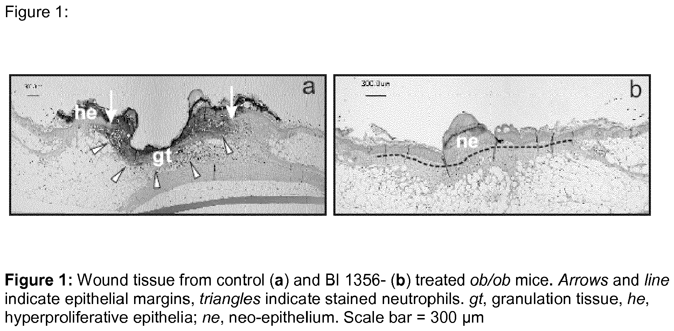

As shown in Figure 1, histological analysis of wound tissues reveals a profound improvement of wound morphology and wound re-epithelialization in BI 1356 treated animals.

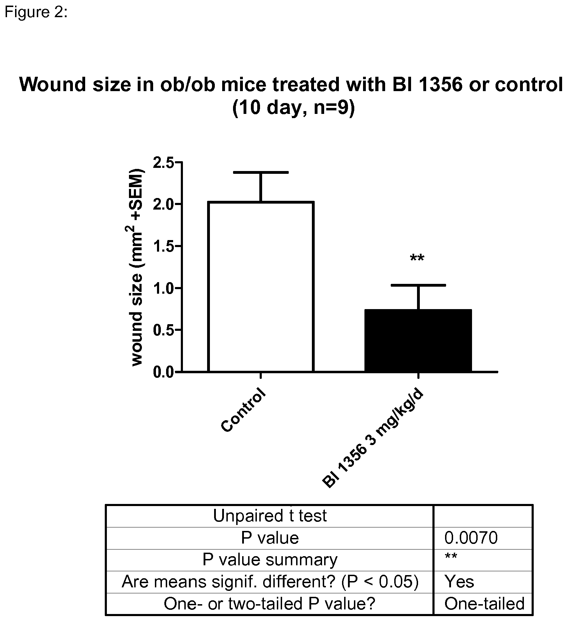

Further as shown in Figure 1, polymorphonuclear neutrophils (PMN) are assessed by immunohistologic analysis of Ly6G (indicated by triangles, Figure 1, thereby reflecting the inflammatory status) as a robust readout for wound inflammation. Neutrophils act as first-line-defence cells in wounds; however in chronic wounds prolonged neutrophil infiltration amplifies inflammation and impairs wound closure. It is observed, in accordance to improved wound epithelialization, an overall reduction of PMN accumulation in BI 1356 treated ob/ob mice, but not in control animals.
A)
B) Summary of wound size in BI 1356 and non-treated ob/ob mice
   As shown in Figure 2, following administration of BI 1356 to ob/ob mice and quantification of the distance between wound margin epithelia demonstrates that BI 1356 treated animals have significantly reduced wound sizes.
C) Glucose homeostatis in ob/ob mice treated with BI 1356 or vehicle

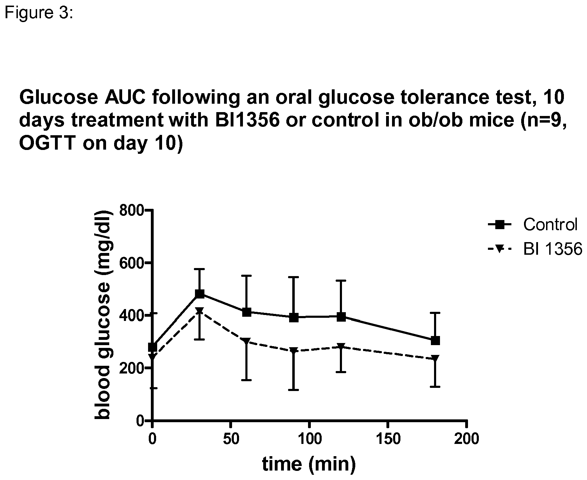

As shown in Figure 3, following administration of BI 1356 to ob/ob mice glucose excursion is decreased.

In accordance to decreased glucose excursion DPP-4 activity is highly significant (p<0.0001) reduced to 80% in ob/ob mice following 12 days treatment with BI 1356 in the dose of 3 mg/kg/d.

In normal C57BI/6 mice (n=10, each group), 14 days treatment in the highest dose of 30 mg/kg/d achieves full DPP-4 inhibition of 95% (p<0.0001). In these animals BI 1356 treatment also demonstrates a tendency in improving wound healing. Estimated half life of wound closure, i.e. time at which 50% of area of the wounds are closed, are calculated to be 7.7 days for controls (n=10) and 6.8 days in BI 1356 treated animals (n=10), however not being statistically significant.

### Brief Description of the Drawings:

Figure 1 shows wound tissue from control (a) and BI 1356- (b) treated *ob*/*ob* mice. *Arrows* and *line* indicate epithelial margins, *triangles* indicate stained neutrophils. *gt*, granulation tissue, *he*, hyperproliferative epithelia; *ne*, neo-epithelium. Scale bar = 300 µm.
Figure 2 shows wound size in ob/ob mice treated with BI 1356 or control (10 day, n = 9).
Figure 3 shows Glucose AUC following an oral glucose tolerance test, 10 days treatment with BI 1356 or control in ob/ob mice (n = 9, OGTT on day 10).

## Claims

1. A DPP-4 inhibitor for use in wound healing in diabetic patients, wherein said DPP-4 inhibitor is selected from the group consisting of
1-[(4-methyl-quinazolin-2-yl)methyl]-3-methyl-7-(2-butyn-1-yl)-8-(3-(*R*)-amino-piperidin-1-yl)-xanthine,
1-[([1,5]naphthyridin-2-yl)methyl]-3-methyl-7-(2-butyn-1-yl)-8-((*R*)-3-amino-piperidin-1-yl)-xanthine,
1-[(quinazolin-2-yl)methyl]-3-methyl-7-(2-butyn-1-yl)-8-((*R*)-3-amino-piperidin-1-yl)-xanthine,
2-((R)-3-amino-piperidin-1-yl)-3-(but-2-ynyl)-5-(4-methyl-quinazolin-2-ylmethyl)-3,5-dihydro-imidazo[4,5-d]pyridazin-4-one,
1-[(3-cyano-quinolin-2-yl)methyl]-3-methyl-7-(2-butyn-1-yl)-8-((*R*)-3-amino-piperidin-1-yl)-xanthine,
1-[(4-methyl-quinazolin-2-yl)methyl]-3-methyl-7-(2-butyn-1-yl)-8-[(*S*)-(2-amino-propyl)-methylamino]-xanthine,
1-[(4,6-dimethyl-pyrimidin-2-yl)methyl]-3-methyl-7-(2-butyn-1-yl)-8-((*R*)-3-amino-piperidin-1-yl)-xanthine and
1-[(quinoxalin-6-yl)methyl]-3-methyl-7-(2-butyn-1-yl)-8-((*R*)-3-amino-piperidin-1-yl)-xanthine,
or a pharmaceutically acceptable salt thereof;
wherein said DPP-4 inhibitor is administered topically.

2. The DPP-4 inhibitor for use according to claim 1, wherein said DPP-4 inhibitor is 1-[(4-methyl-quinazolin-2-yl)methyl]-3-methyl-7-(2-butyn-1-yl)-8-(3-(*R*)-amino-piperidin-1-yl)-xanthine.

3. The DPP-4 inhibitor for use according to any one of the claims 1 to 2 for improving wound epithelialization.

4. The DPP-4 inhibitor for use according to any one of the claims 1 to 2 for promoting neo-epithelialization.

5. The DPP-4 inhibitor for use according to any one of the claims 1 to 2 for promoting tissue regeneration.

6. The DPP-4 inhibitor for use according to any one of the claims 1 to 2 for diminishing wound size.

7. The DPP-4 inhibitor for use according to any one of the claims 1 to 2 for reducing destructive wound inflammation, such as e.g. for reducing the number of polymorphonuclear neutrophils (PMN).

8. The DPP-4 inhibitor for use according to any one of the claims 1 to 2 for treating and/or preventing wound healing deficit or impairments in the wound healing process in diabetic patients.

9. A topical preparation for use in wound healing in diabetic patients, said topical preparation comprising a DPP-4 inhibitor selected from the group consisting of
1-[(4-methyl-quinazolin-2-yl)methyl]-3-methyl-7-(2-butyn-1-yl)-8-(3-(*R*)-amino-piperidin-1-yl)-xanthine,
1-[([1,5]naphthyridin-2-yl)methyl]-3-methyl-7-(2-butyn-1-yl)-8-((*R*)-3-amino-piperidin-1-yl)-xanthine,
1-[(quinazolin-2-yl)methyl]-3-methyl-7-(2-butyn-1-yl)-8-((*R*)-3-amino-piperidin-1-yl)-xanthine,
2-((R)-3-amino-piperidin-1-yl)-3-(but-2-ynyl)-5-(4-methyl-quinazolin-2-ylmethyl)-3,5-dihydro-imidazo[4,5-d]pyridazin-4-one,
1-[(3-cyano-quinolin-2-yl)methyl]-3-methyl-7-(2-butyn-1-yl)-8-((*R*)-3-amino-piperidin-1-yl)-xanthine,
1-[(4-methyl-quinazolin-2-yl)methyl]-3-methyl-7-(2-butyn-1-yl)-8-[(*S*)-(2-amino-propyl)-methylamino]-xanthine,
1-[(4,6-dimethyl-pyrimidin-2-yl)methyl]-3-methyl-7-(2-butyn-1-yl)-8-((*R*)-3-amino-piperidin-1-yl)-xanthine and
1-[(quinoxalin-6-yl)methyl]-3-methyl-7-(2-butyn-1-yl)-8-((*R*)-3-amino-piperidin-1-yl)-xanthine,
or a pharmaceutically acceptable salt thereof;
with suitable carrier materials for topical preparations.

10. The topical preparation for use according to claim 9, which is an ointment.

11. The topical preparation for use according to claim 9 or 10, wherein the carrier materials for topical preparations are selected from glycerides, semi- synthetic and synthetic glycerides, hydrogenated oils, liquid waxes, liquid paraffins, liquid fatty alcohols, sterols, polyethylene glycols and/or cellulose derivatives.

12. The DPP-4 inhibitor for use according to any one of the claims 1 to 8 in combination with one or more other therapeutically active agents selected from metformin, pioglitazone and telmisartan, for separate, sequential, simultaneous, concurrent or chronologically staggered use in wound healing.

13. The DPP-4 inhibitor for use according to any one of the claims 1 to 8, wherein said DPP-4 inhibitor exerts direct favourable (e.g. extraglycemic) effects on the wound repairing process in a type 2 diabetic subject.

14. The DPP-4 inhibitor for use according to any one of the claims 1 to 8, wherein said DPP-4 inhibitor is suitable for providing additional therapeutic benefits to diabetic patients with or at risk of skin diseases, wounds and/or wound healing disturbances or impairments beyond improving glycemic control.

## Patentansprüche

1. DPP-4-Inhibitor zur Verwendung in der Wundheilung von Diabetes-Patienten, wobei besagter DPP-4-Inhibitor ausgewählt ist aus der Gruppe, bestehend aus
1-[(4-Methyl-chinazolin-2-yl)methyl]-3-methyl-7-(2-butin-1-yl)-8-(3-(*R*)-amino-piperidin-1-yl)-xanthin,
1-[([1,5]Naphthyridin-2-yl)methyl]-3-methyl-7-(2-butin-1-yl)-8-((*R*)-3-amino-piperidin-1-yl)-xanthin,
1-[(Chinazolin-2-yl)methyl]-3-methyl-7-(2-butin-1-yl)-8-((*R*)-3-amino-piperidin-1-yl)-xanthin,
2-((R)-3-Amino-piperidin-1-yl)-3-(but-2-inyl)-5-(4-methyl-chinazolin-2-ylmethyl)-3,5-dihydro-imidazo[4,5-d]pyridazin-4-on,
1-[(3-Cyano-chinolin-2-yl)methyl]-3-methyl-7-(2-butin-1-yl)-8-((*R*)-3-amino-piperidin-1-yl)-xanthin,
1-[(4-Methyl-chinazolin-2-yl)methyl]-3-methyl-7-(2-butin-1-yl)-8-[(*S*)-(2-amino-propyl)-methylamino]-xanthin,
1-[(4,6-Dimethyl-pyrimidin-2-yl)methyl]-3-methyl-7-(2-butin-1-yl)-8-((*R*)-3-amino-piperidin-1-yl)-xanthin und
1-[(Chinoxalin-6-yl)methyl]-3-methyl-7-(2-butin-1-yl)-8-((*R*)-3-amino-piperidin-1-yl)-xanthin,
oder einem pharmazeutisch annehmbaren bzw. akzeptablen Salz hiervon;
wobei besagter DPP-4-Inhibitor topisch verabreicht wird.

2. DPP-4-Inhibitor zur Verwendung gemäß Anspruch 1, wobei besagter DPP-4-Inhibitor 1-[(4-Methyl-chinazolin-2-yl)methyl]-3-methyl-7-(2-butin-1-yl)-8-(3-(*R*)-amino-piperidin-1-yl)-xanthin ist.

3. DPP-4-Inhibitor zur Verwendung gemäß irgendeinem der Ansprüche 1 bis 2 zur Verbesserung der Wund-Epithelisation.

4. DPP-4-Inhibitor zur Verwendung gemäß irgendeinem der Ansprüche 1 bis 2 zur Förderung der Neu-Epithelisation.

5. DPP-4-Inhibitor zur Verwendung gemäß irgendeinem der Ansprüche 1 bis 2 zur Förderung der Geweberegeneration.

6. DPP-4-Inhibitor zur Verwendung gemäß irgendeinem der Ansprüche 1 bis 2 zur Verringerung der Wundgröße.

7. DPP-4-Inhibitor zur Verwendung gemäß irgendeinem der Ansprüche 1 bis 2 zur Reduzierung der destruktiven Wundentzündung, wie z.B. zur Reduzierung der Zahl der polymorph-nuklearen Neutrophile (PMN).

8. DPP-4-Inhibitor zur Verwendung gemäß irgendeinem der Ansprüche 1 bis 2 zur Behandlung und/oder Vorbeugung eines Wundheilungsdefizits oder einer Wundheilungsstörung im Wundheilungsprozess von Diabetes-Patienten.

9. Topische Zubereitung zur Verwendung in der Wundheilung von Diabetes-Patienten, wobei besagte topische Zubereitung einen DPP-4-Inhibitor umfasst, ausgewählt aus der Gruppe, bestehend aus
1-[(4-Methyl-chinazolin-2-yl)methyl]-3-methyl-7-(2-butin-1-yl)-8-(3-(*R*)-amino-piperidin-1-yl)-xanthin,
1-[([1,5]Naphthyridin-2-yl)methyl]-3-methyl-7-(2-butin-1-yl)-8-((*R*)-3-amino-piperidin-1-yl)-xanthin,
1-[(Chinazolin-2-yl)methyl]-3-methyl-7-(2-butin-1-yl)-8-((*R*)-3-amino-piperidin-1-yl)-xanthin,
2-((R)-3-Amino-piperidin-1-yl)-3-(but-2-inyl)-5-(4-methyl-chinazolin-2-ylmethyl)-3,5-dihydro-imidazo[4,5-d]pyridazin-4-on,
1-[(3-Cyano-chinolin-2-yl)methyl]-3-methyl-7-(2-butin-1-yl)-8-((*R*)-3-amino-piperidin-1-yl)-xanthin,
1-[(4-Methyl-chinazolin-2-yl)methyl]-3-methyl-7-(2-butin-1-yl)-8-[(*S*)-(2-amino-propyl)-methylamino]-xanthin,
1-[(4,6-Dimethyl-pyrimidin-2-yl)methyl]-3-methyl-7-(2-butin-1-yl)-8-((*R*)-3-amino-piperidin-1-yl)-xanthin und
1-[(Chinoxalin-6-yl)methyl]-3-methyl-7-(2-butin-1-yl)-8-((*R*)-3-amino-piperidin-1-yl)-xanthin,
oder ein pharmazeutisch annehmbares bzw. akzeptables Salz hiervon;
mit geeigneten Trägermaterialien für topische Zubereitungen.

10. Topische Zubereitung zur Verwednung gemäß Anspruch 9, welche eine Salbe ist.

11. Topische Zubereitung zur Verwendung gemäß Anspruch 9 oder 10, wobei die Trägermaterialien für topische Zubereitungen ausgewählt sind aus Glyceriden, halbsynthetischen und synthetischen Glyceriden, gehärteten Ölen, flüssigen Wachsen, flüssigen Paraffinen, flüssigen Fettalkoholen, Sterolen, Polyethylenglykolen und/oder Zellulosederivaten.

12. DPP-4-Inhibitor zur Verwendung gemäß irgendeinem der Ansprüche 1 bis 8 in Kombination mit einem oder mehreren anderen therapeutisch aktiven Mitteln, ausgewählt aus Metformin, Pioglitazon und Telmisartan, zur voneinander getrennten, aufeinander folgenden, simultanen, gleichzeitigen oder zeitlich gestaffelten Verwendung in der Wundheilung.

13. DPP-4-Inhibitor zur Verwendung gemäß irgendeinem der Ansprüche 1 bis 8, wobei besagter DPP-4-Inhibitor bei einer Person mit Typ 2-Diabetes eine direkte, günstige (z.B. extraglykämische) Wirkung auf den Wundheilungsprozess ausübt.

14. DPP-4-Inhibitor zur Verwendung gemäß irgendeinem der Ansprüche 1 bis 8, wobei besagter DPP-4-Inhibitor dazu geeignet ist, Diabetes-Patienten mit oder mit einem Risiko von Hautkrankheiten, Wunden und/oder Wundheilungsstörungen oder -beeinträchtigungen über die Verbesserung der Blutzuckerkontrollen hinaus zusätzliche therapeutische Vorteile zu bieten.

## Revendications

1. Inhibiteur de la DPP-4 pour son utilisation dans la cicatrisation des plaies chez les patients diabétiques, dans lequel ledit inhibiteur de la DPP-4 est choisi dans le groupe consistant en
1-[(4-méthyl-quinazolin-2-yl)méthyl]-3-méthyl-7-(2-butin-1-yl)-8-(3-(*R*)-amino-pipéridin-1-yl)-xanthine,
1-[([1,5]naphthyridin-2-yl)méthyl]-3-méthyl-7-(2-butin-1-yl)-8-((*R*)-3-amino-pipéridin-1-yl)-xanthine,
1-[(quinazolin-2-yl)méthyl]-3-méthyl-7-(2-butin-1-yl)-8-((*R*)-3-amino-pipéridin-1-yl)-xanthine,
2-((R)-3-amino-pipéridin-1-yl)-3-(but-2-inyl)-5-(4-méthyl-quinazolin-2-ylméthyl)-3,5-dihydro-imidazo[4,5-d]pyridazin-4-one,
1-[(3-cyano-quinolin-2-yl)méthyl]-3-méthyl-7-(2-butin-1-yl)-8-((*R*)-3-amino-pipéridin-1-yl)-xanthine,
1-[(4-méthyl-quinazolin-2-yl)méthyl]-3-méthyl-7-(2-butin-1-yl)-8-[(*S*)-(2-amino-propyl)-méthylamino]-xanthine,
1-[(4,6-diméthyl-pyrimidin-2-yl)méthyl]-3-méthyl-7-(2-butin-1-yl)-8-((*R*)-3-amino-pipéridin-1-yl)-xanthine et
1-[(quinoxalin-6-yl)méthyl]-3-méthyl-7-(2-butin-1-yl)-8-((*R*)-3-amino-pipéridin-1-yl)-xanthine,
ou un sel pharmaceutiquement acceptable de ceux-ci;
dans lequel ledit inhibiteur de la DPP-4 est administré topiquement.

2. L'inhibiteur de la DPP-4 pour son utilisation selon la revendication 1, dans lequel ledit inhibiteur de la DPP-4 est 1-[(4-méthyl-quinazolin-2-yl)méthyl]-3-méthyl-7-(2-butin-1-yl)-8-(3-(*R*)-amino-pipéridin-1-yl)-xanthine.

3. L'inhibiteur de la DPP-4 pour son utilisation selon l'une quelconque des revendications 1 à 2 pour l'amélioration de l' épithélialisation des plaies.

4. L'inhibiteur de la DPP-4 pour son utilisation selon l'une quelconque des revendications 1 à 2 pour la promotion de la néo-épithélialisation.

5. L'inhibiteur de la DPP-4 pour son utilisation selon l'une quelconque des revendications 1 à 2 pour la promotion de la régénération du tissu.

6. L'inhibiteur de la DPP-4 pour son utilisation selon l'une quelconque des revendications 1 à 2 pour la réduction de la taille des plaies.

7. L'inhibiteur de la DPP-4 pour son utilisation selon l'une quelconque des revendications 1 à 2 pour la réduction de l'inflammation destructive des plaies, comme par exemple pour la réduction du nombre des neutrophiles polymorphonucléaire (PMN).

8. L'inhibiteur de la DPP-4 pour son utilisation selon l'une quelconque des revendications 1 à 2 dans le traitement et/ou la prévention de la cicatrisation insuffisante ou troublée pendant le processus de guérison chez les patients diabétiques.

9. Préparation topique pour son utilisation dans la cicatrisation (des plaies) chez les patients diabétiques, ladite préparation topique comprenant un inhibiteur de la DPP-4 choisi dans le groupe consistant en
1-[(4-méthyl-quinazolin-2-yl)méthyl]-3-méthyl-7-(2-butin-1-yl)-8-(3-(*R*)-amino-pipéridin-1-yl)-xanthine,
1-[([1,5]naphthyridin-2-yl)méthyl]-3-méthyl-7-(2-butin-1-yl)-8-((*R*)-3-amino-pipéridin-1-yl)-xanthine,
1-[(quinazolin-2-yl)méthyl]-3-méthyl-7-(2-butin-1-yl)-8-((*R*)-3-amino-pipéridin-1-yl)-xanthine,
2-((R)-3-amino-pipéridin-1-yl)-3-(but-2-inyl)-5-(4-méthyl-quinazolin-2-ylméthyl)-3,5-dihydro-imidazo[4,5-d]pyridazin-4-one,
1-[(3-cyano-quinolin-2-yl)méthyl]-3-méthyl-7-(2-butin-1-yl)-8-((*R*)-3-amino-pipéridin-1-yl)-xanthine,
1-[(4-méthyl-quinazolin-2-yl)méthyl]-3-méthyl-7-(2-butin-1-yl)-8-[(*S*)-(2-amino-propyl)-méthylamino]-xanthine,
1-[(4,6-diméthyl-pyrimidin-2-yl)méthyl]-3-méthyl-7-(2-butin-1-yl)-8-((*R*)-3-amino-pipéridin-1-yl)-xanthine et
1-[(quinoxalin-6-yl)méthyl]-3-méthyl-7-(2-butin-1-yl)-8-((*R*)-3-amino-pipéridin-1-yl)-xanthine,
ou un sel pharmaceutiquement acceptable de ceux-ci;
avec un support adapté aux préparations topiques.

10. Composition topique pour son utilisation selon la revendication 9, qui est une pommade.

11. Composition topique pour son utilisation selon la revendication 9 ou 10, dans lequel les supports pour les préparations topiques sont choisis parmi les glycérides, les glycérides semi-synthétiques et synthétiques, les huiles hydrogénées, les cires liquides, les paraffines liquides, les alcools gras liquides, les stérols, les polyéthylèneglycols et/ou les dérivées de la cellulose.

12. L'inhibiteur de la DPP-4 pour son utilisation selon l'une quelconque des revendications 1 à 8 en combinaison avec un ou plusieurs autres agents thérapeutiquement actifs choisis parmi la metformine, la pioglitazone et le telmisartan, pour l'utilisation séparée, séquentielle, simultanée, concomitante ou échelonnée dans le temps dans la cicatrisation des plaies.

13. L'inhibiteur de la DPP-4 pour son utilisation selon l'une quelconque des revendications 1 à 8, dans lequel ledit inhibiteur de la DPP-4 exerce des effets (p. ex. extraglycémique) favorables directes sur le processus de guérison chez un patient diabétique de type 2.

14. L'inhibiteur de la DPP-4 pour son utilisation selon l'une quelconque des revendications 1 à 8, dans lequel ledit inhibiteur de la DPP-4 est apte à procurer des avantages thérapeutiques supplémentaires au-delà de l' amélioration du contrôle glycémique aux patients diabétiques ayant ou étant en risque de maladies de la peau, des plaies et/ou des cicatrisations insuffisantes ou troublées.
